# EUROPEAN PATENT SPECIFICATION

(11) **EP 1 691 747 B1**
(45) Date of publication and mention of the grant of the patent: **23.05.2012**
(21) Application number: 04819131.6
(22) Date of filing: 15.11.2004
(51) Int. Cl.: A61F 13/00

(54) **CONTROL OF CARDIAC ARRHYTHMIAS BY MODIFICATION OF NEURONAL CONDUCTION WITHIN FAT PADS OF THE HEART**
KONTROLLE VON HERZARRYTHMIEN DURCH VERÄNDERUNG DER NEURONALEN LEITUNG IN FETTPOLSTERN DES HERZENS
REGULATION DES ARYTHMIES CARDIAQUES PAR MODIFICATION DE LA CONDUCTION NEURONALE DANS LES COUSSINETS ADIPEUX DU COEUR

(30) Priority: 13.11.2003 US 519588 P; 20.11.2003 US 523848 P; 03.03.2004 US 550185 P; 04.03.2004 US 550076 P
(43) Date of publication of application: 23.08.2006
(73) Proprietor: CardioPolymers, Inc., Laguna Hills, CA 92653 (US)
(72) Inventor: MACIEJEWSKI, Mark, Edina, MN 55436 (US); PETERS, Nicholas, S., Farnham Common Bucks SL2 3EW (GB); MAZGALEV, Todor N., Cleveland, OH 44124 (US)
(74) Representative: Viering, Jentschura & Partner
(86) International application number: PCT/US2004/038128
(87) International publication number: WO 2005/049111

(56) References cited:
- WO-A-99/60923
- WO-A-03/026480
- US-B1- 6 292 695
- US-B1- 6 292 695
- OZ M.C. ET AL: 'Controlled Clinical Trial of a Novel Hemostatic Agent in Cardiac Surgery' ANNALS OF THORACIC SURGERY vol. 69, no. 5, May 2000, pages 1376 - 1382, XP002995095

## Description

### BACKGROUND OF THE INVENTION

Field of the Invention

The present invention relates to the treatment of medical conditions associated with the heart, and more particularly to control of cardiac arrhythmias by modification of neuronal conduction within the fat pads of the heart.

Description of the Related Art

The autonomic nervous system (ANS) is divided into sympathetic and parasympathetic systems. The sympathetic system increases the heart rate and ventricular contraction, dilates the blood vessels in skeletal muscles, constricts blood vessels in the skin and guts, increases blood sugar level, stimulates sweating, dilates the pupils, inhibits activities of the guts and gastric secretion. The parasympathetic system is more active at rest, having in general anabolic effects. For example, it slows down the heart rate, constricts the pupils, increases gastric secretion and intestinal motility.

Neural control of the heart is dependent on the levels of activity of sympathetic and parasympathetic neurons and the interactions that occur between these two limbs of the autonomic nervous system. As disclosed in McGuirt, A.S., Autonomic interactions for control of atrial rate are maintained after SA nodal parasympathectomy, Am. J. Physiol. 272 (Heart Circ. Physiol. 41), 1997, H2525-H2533, for control of regional cardiac function, both pre- and post- junctional interactions occur between the separate autonomic projections to the heart, particularly at the end-organ target sites such as the SA node, the AV node, and contractile elements of the atria and ventricles. Cardiac ganglia contain parasympathetic, sympathetic, and afferent neurons. In the normal physiologic process, heart conduction moves from cell to cell, from the SA node to AV node, and from the atrium to the ventricles.

Cardiac arrhythmias are abnormal conditions associated with the various chambers and other structures of the heart. Atrial fibrillation ("AF") is the most frequently occurring sustained cardiac arrhythmia, particularly among the elderly and among patients with organic heart disease, as well as among patients recovering from coronary artery bypass graft ("CABG") surgery; see Steinberg, Jonathan S., Postoperative Atrial Fibrillation: A Billion-Dollar Problem, Journal of the American College of Cardiology, Vol. 43, No. 6, 2004. AF occurs in, for example, as many as 50% of patients undergoing cardiac operations. Patients with chronic AF have symptomatic tachycardia or low cardiac output and have a 5-10% risk of thromboembolic complications and events.

A common treatment for AF is cardioversion, alone or in combination with anti-arrhythmic therapy, to restore sinus rhythm. Recurrence rates after such therapy as high as 75% have been reported. Pharmacologic therapy is associated with adverse effects in a significant proportion of patients with AF. Other more current conventional methods of treating AF center around ablation (destruction) of the aberrant conduction pathways, either through a surgical approach or by use of various forms of energy to ablate conduction to electrically isolate discrete atrial regions.

Ablation is generally a treatment technique intended to destructively create conduction blocks to intervene and stop aberrant conduction pathways that otherwise disturb the normal cardiac cycle. Typical ablation technology for forming conduction blocks uses systems and methods designed to kill tissue at the arrhythmogenic source or along an aberrant, cascading conductive pathway. Typically, cells in the conductive pathway are destroyed via hyperthermia, hypothermia, or chemical action. Suitable types of energy to induce hyperthermia include radiofrequency electrical current and ultrasound, microwave, and laser energy. Hypothermia may be induced using cryotherapy. An example of chemical ablation is destructive ethanol delivery to cardiac tissue. Despite the significant benefits and successful treatments that have been observed by creating conduction blocks using various of these techniques, each is associated with certain adverse consequences. For example, ablative hyperthermia or other modes causing necrosis have been observed to result in scarring, thrombosis, collagen shrinkage, and undesired structural damage to deeper tissues.

Therefore, there is a need for control of cardiac arrhythmias without ablating cardiac tissue.

### BRIEF SUMMARY OF THE INVENTION

One embodiment of the present invention relates to an agent which is effective for modifying conduction of impulses in ganglionic synapses (130) in a cardiac fat pad (140) and substantially non-ablative to the ganglionic synapses (130) in the cardiac fat pad (140), wherein the agent is a biopolymer, fibroblasts, a neurotoxin, or a growth factor, for use in a method for treating cardiac arrhythmia in a heart of a patient, wherein the method comprises injection of a therapeutically effective amount of the agent into at least one cardiac fat pad (140) in proximity to the ganglionic synapses (130).

### BRIEF DESCRIPTION OF THE SEVERAL VIEWS OF THE DRAWINGS

FIG. 1 is a schematic view of a nerve pathway from pre-ganglionic neurons in the cervical vagus to the sinus node of a heart.

FIG. 2 is a graph showing in the time domain the results after a period of time of injecting fibrin glue into the sinus node fat pad of a canine test subject.

FIG. 3 is a schematic view of an catheter injection system for a single component conduction-modification agent.

FIG. 4 is a transverse cross sectional view of a catheter in which a needle is slideably housed in a single lumen shaft within the catheter body.

FIG. 5 is a transverse cross sectional view of a catheter that has three lumens, one of which slideably houses a needle.

FIG. 6 is a transverse cross sectional view of a catheter that has four lumens, which slideably house a needle, a pull-wire, and two lead wires.

FIG. 7 is a schematic view of an illustrative catheter injection system for a dual component conduction-modifying agent.

FIG. 8 is a transverse cross sectional view of a catheter that has two lumens which slideably house respective needles.

FIG. 9 is a schematic view of an illustrative injection system for injecting a dual component conduction-modifying agent.

FIG. 10 is a schematic view of another illustrative injection system for injecting a dual component conduction-modifying agent.

FIG. 11 shows a needle having a body that is sectioned with a partition to form separate and distinct lumens.

FIG. 12 shows a needle having a body within which two separate and distinct lumens are formed.

FIG. 13 shows a needle having a body that surrounds a second inner body to form separate and distinct lumens.

FIG. 14 is a graph showing in the time domain the impact on sinus rhythm of SN fat pad simulation before fibrin glue injection.

FIG. 15 is a graph showing in the time domain the impact on sinus rhythm of SN fat pad simulation immediately after fibrin glue injection.

FIG. 16 is a graph showing in the time domain the impact on atrial pacing of AVN fat pad simulation before fibrin glue injection.

FIG. 17 is a graph showing in the time domain the impact on atrial pacing of AVN fat pad simulation immediately after fibrin glue injection.

FIG. 18 is a graph showing in the time domain the impact on atrial fibrillation of AVN fat pad simulation before fibrin glue injection.

FIG. 19 is a graph showing in the time domain the impact on atrial fibrillation of AVN fat pad simulation immediately after fibrin glue injection.

### DETAILED DESCRIPTION OF THE INVENTION, INCLUDING THE BEST MODE

The two broad strategic treatment options for atrial fibrillation ("AF") are rhythm control and rate control. For rhythm control, treatment is directed toward restoring and maintaining the sinus rhythm. The pulmonary veins and atria have rich autonomic innervation, largely via cardiac ganglia that exist in fat pads in various well defined pericardial locations, some adjacent to the pulmonary veins. It has long been recognized that autonomic manipulation and intervention can dramatically alter the threshold for AF induction and persistence, and this approach has in the past been used experimentally to create appropriate models of AF.

Emerging data from clinical trials based on strategies for PV isolation indicate that clinical success may be possible without achieving complete isolation. These observations indicate that what is being achieved is not only isolation of the triggers for AF, but also modification of the substrate by ablation of autonomic innervation. It is possible that the latter effect is the predominant determinant of therapeutic success.

Various researchers have reported that experimental interference with the cardiac autonomic ganglia can achieve modification of tendency to AF, and early clinical studies ablating around the mouths of pulmonary vei ns by targeting sites at which stimulation produces measurable changes in autonomic tone, indicating sites of autonomic innervation downstream from the cardiac ganglia, have shown success in abolishing AF. We believe that a mechanism for AF is dependent on local autonomic dysfunction (dysautonomia), and that the AF triggers and substrate may be treated by specific modification of function of the autonomic innervation, or interruption of the autonomic supply, or both at the level of the autonomic ganglia within the fat pads.

We have found that the AF triggers and especially the substrate may be treated by modifying neuronal conduction in various epicardial fat pads of the heart. The ganglia of principal interest are in three epicardia I fat pads: the right pulmonary ("RPV") fat pad, which supplies nerve fibers preferentially to the superior right atrium and sinus node; the inferior vena cava-left arterial ("IVC-LA") fat pad, which supplies nerve fibers to the AV node region and both atria; and a third fat pad ("SVC-AO") located between the superior vena cava and aorta. The SVC-AO fat pad provides efferent fibers to both the RPV and IVC-LA fat pads as well as additional fibers to both atria. These fat pads are of particular interest because they are accessible and distinctly identifiable, although other fat pads may be suitable as well. Of these three, the RPV fat pad and the IVC-LA fat pad are particularly preferred since efferent fibers from the SVC-AO fat pad are provided to them as well.

Various conduction-modifying agents include various biopolymers such as, for example, fibrin glue and alginate, various cells such as, for example, fibroblasts (allogeneic or autologous), various neurotoxins such as, for example, Botulinum Type A, and various growth factors such as, for example, fibroblast growth factor. The conduction-modifying agents are introduced either epicardially or endocardially to the fat pads in any desired manner, preferably by injection from a catheter inserted percutaneously or by direct injection through the epicardial as in open heart surgery. Preferably the patient's heart is beating throughout the Injection.

The conduction-modifying agent may be, for example, fibrin glue formed from a one-to-one (1:1) ratio mixture of fibrinogen precursor to thrombin precursor. The fibrinogen and thrombin preferably are delivered separately to the targeted anatomical location in unmixed form via a dual channeled needles or separate needles, so that mixing occurs at the targeted anatomical location and not within the delivery system or outside of the targeted anatomical location. A satisfactory dose for a positive clinical result is a single 1 milliliter fibrin injection into the targeted anatomical location, although the dose may be varied as needed to achieve the desired therapeutic effect.

The conduction-modifying agent may be, for example, fibroblast cells which are injected into a human patient's heart. The fibroblast cells may be injected in a solution of Bovine Serum Albumin ("BSA") or any other appropriate carrier solution that is biocompatable with human tissue. The volume of the injected solution may range in volumes from about 0.1 milliliters up to about 5 milliliters per injection, with as many as 10 million to 100 million fibroblast cells per injection. Multiple injections of fibroblasts may be delivered into the same anatomical location, either during the same medical treatment or over different medical treatments. For example, a "dose" of fibroblasts may be initially delivered to the treatment site with an appropriate "wait and see" designated period to assess clinical efficacy. Then, if deemed appropriate, additional fibroblasts may be injected into the same general anatomical location to augment the initial dosage to yield the desired clinical results. As many as 50 fibroblast injections or more may be injected into the same general anatomical location to yield the desired clinical results.

With further respect to cell delivery, they may be cultured from the patient's own cells (e.g. autologous), or may be foreign to the body such as from a regulated cell culture.

In one particular and illustrative implementation of a cardiac delivery system, a cardiac arrhythmia is treated by delivering a conduction-modifying agent according to the appended claims into one or more cardiac fat pads. A source of the conduction-modifying agent is provided. The cardiac delivery system is coupled to the source to deliver a volume of the conduction-modifying agent from the source to the desired location in the cardiac fat pad.

In an illustrative endocardial implementation of the cardiac delivery system, a cardiac conducting mapping system is included for identifying the source and/or location of a cardiac arrhythmia. The mapping may be performed in any suitable manner, such as, for example, by applying electromagnetic energy or by detecting electrical potentials within the tissue. A material source contains a preparation of a conduction-modifying agent. A catheter is used to deliver the conduction-modifying agent to a fat pad containing innervation associated with the arrhythmia, to modify conduction in the ganglia and thereby reduce or eliminate the arrhythmia. The catheter is adapted to be injected into the fat pad.

In another illustrative implementation of the cardiac delivery system, the material source is a preparation of a dual (or multiple) precursor conduction-modifying agent, and a catheter is provided for delivering the conduction-modifying agent to a fat pad containing innervation associated with the a rrhythmia, to modify conduction in the ganglia and thereby reduce or eliminate the arrhythmia. Separate syringes are used for each of the precursors, and are connected to a branch section that in turn is connected to a multi-channeled catheter. Separate channels extend from each syringe through the branch section and to the end of the catheter. As the plungers of the syringes are depressed, the precursors are carried in their respective separate channels and mix in the fat pad in proximity to ganglionated plexuses immediately after clearing the catheter opening. The catheter is adapted to be injected into the fat pad with its end in proximity to the ganglionated plexuses.

An illustrative implementation of a method for assembling a cardiac arrhythmia treatment system, a delivery catheter is chosen that is capable of delivering a preparation of conduction-modifying agent into a cardiac tissue site such as a fat pad. The delivery catheter is inserted into the tissue, and is also coupled to a source of the conduction-modifying agent.

In a variation of the method of assembly, an injector is included in the delivery catheter for injecting the conduction-modifying agent to the desired fat pad site via the delivery catheter.

Another illustrative implementation of a system for treating cardiac arrhythmia in a patient includes a cardiac delivery system and a source of conduction-modifying agent coupled to the cardiac delivery system. The cardiac delivery system is adapted to deliver the conduction-modifying agent from the source and substantially to a fat pad associated with the patient's heart. The cardiac delivery system may be either epicardial or endocardial, and the conduction-modifying agent may be delivered directly by the delivery system as during an open heart surgical procedure, or may be delivered with a percutaneous translumenal delivery approach. Specifically, delivery may be by a transthoracic minimally invasive technique, or transvascularly (for example, via the coronary sinus or the septal perforators), according to further appropriate device and method variations, respectively.

In one variation of this system, the cardiac delivery system further includes a contact member that is adapted to substantially contact the fat pads, and the cardiac delivery system delivers the conduction-modifying agent to the contact member when it is substantially contacting the fat pads.

In another variation of this system, the cardiac delivery system includes a plurality of needles cooperating with the contact member. The plurality of needles are position by the cardiac delivery system into and substantially throughout the fat pads, so as to inject the fibroblast cells substantially into and throughout the fat pads for modifying ganglia conduction.

Various materials are useful as the conduction-modifying agent. One such material is a composition that comprises a scaffold from fibrin glue or other biopolymer agent combined with fibroblasts and/or neurotoxin and/or growth factor. Optionally the composition comprises only (1) a scaffold from fibrin glue or other biopolymer agent, (2) fibroblasts, (3) neurotoxin, or (4) growth factor that blocks or impairs conduction in the fat pad ganglia.

In one implementation, the conduction-modifying agent includes autologous fibroblasts. Fibroblasts are nonconductive type of cell, and also secrete collagen, which acts as an electrical insulator. The autologous fibroblasts are derived from a biopsy of a patient's skin, amplified, and injected and/or grafted. In another implementation, such fibroblasts are removed from the patient and prepared in a manner so that they are suitable for delivery to the desired region of the heart. The preparation is coupled to an appropriate delivery catheter.

Principles of Conduction Modification in Ganglia of the Cardiac Fat Pads

Mammalian hearts have various collections of ganglia, known as ganglionated plexuses, associated with nerves. The ganglia contain many intrinsic neurons, most of which are multipolar, although some unipolar and bipolar neurons are also present. In the human heart, intrinsic cardiac ganglia and their associated nerves are found primarily embedded in epicardial fat, in which they form five atrial and five ventricular ganglionated plexuses. As disclosed in Armour, J. Andrew, et al., Gross and Microscopic Anatomy of the Human Intrinsic Cardiac Nervous System, The Anatomical Record, Vol. 247, 1997, pp. 289-298, atrial ganglionated plexuses ("AGP") may be found on the superior surface of the right atrium (the superior right AGP), the superior surface of the left atrium (the superior left AGP), the posterior surface of the right atrium (the posterior right AGP), the posterior medial surface of the left atrium (the posteromedial left AGP) (the posterior right AGP and the posteromedial left AGP fuse medially where they extend anteriorly into the interatrial septum), and the inferior and lateral aspect of the posterior left atrium (the posterolateral left AGP); while ventricular ganglionated plexuses ("VGP") may be found in fat surrounding the aortic root (the aortic root VGP, with right, anterior, left and posterior components), at the origins of the right and left coronary arteries, the latter extending to the origins of the left anterior descending and circumflex coronary arteries (the anterior descending VGP), at the origin of the posterior descending coronary artery (the posterior descending VGP), adjacent to the origin of the right acute marginal coronary artery (the right acute marginal VGP), and at the origin of the left obtuse marginal coronary artery (the obtuse marginal VGP). Neurons may also be located outside these sites, primarily in fat associated with branch points of other large coronary arteries.

While other fat pads may receive treatment in accordance with the principles described herein, three epicardial fat pads are of principal interest. They are the right pulmonary ("RPV") fat pad which supplies nerve fibers preferentially to the superior right atrium and the sinus node, the inferior vena cava-left arterial ("IVC-LA") fat pad which supplies nerve fibers to the AV node region and both atria, and the superior vena cava-aorta ("SVC-AO") fat pad which supplies efferent fibers to both the RPV and IVC-LA fat pads as well as additional fibers to both atria.

Within the ganglionated plexuses, impulses are conducted from one neuron to another at sites of functional apposition between neurons known as synapses. Although a few synapses in the central nervous system are electrical synapses, conduction between neurons is usually by a chemical neurotransmitter released by the axon terminal of the excited or presynaptic cell. The neurotransmitter diffuses across the synaptic cleft to bind with receptors on the postsynaptic cell membrane, which effects electrical changes in the postsynaptic cell.

One type of conduction-modifying agent is an injectable biopolymer of which fibrin glue and alginate are examples. The biopolymer becomes a semi-rigid scaffold upon injection, forming a fibrin matrix which we believe mechanically disrupts the conduction of impulses in the synapses. The fibrin itself is electrically insulating and would be expected to inhibit conduction in any electrical synapses that may be present.

A typical fibrin matrix has an interesting property that makes it particularly advantageous for the control of AF in patients recovering from coronary artery bypass graft ("CABG") surgery and other cardiac surgery and procedures. As observed in Steinberg, Jonathan S., Editorial Comment: Postoperative Atrial Fibrillation, A Billion Dollar Problem, Journal of the American College of Cardiology, Vol. 43, No. 6, March 17, 2004, pp. 1001-1003, AF is the most common complication associated with coronary artery bypass graft ("CABG") surgery. AF clusters tightly in the first two to four days after surgery. The clustering is in part the result of preexisting electrophysiologic vulnerability in the atria, but a number of contributing factors are likely to be present, along with preoperative electrical and structural abnormality as well as postoperative profibrillatory factors. As reported by Cummings, Jennifer E., Preservation of the anterior fat pad paradoxically decreases the incidence of postoperative atrial fibrillation in humans, Journal of the America College of Cardiology, Vol. 43, 2004, pp. 994-1000, the common practice of removing the anterior fat pad during CABG appears to be proarrhythmic, and may be due to upset of the balance of sympathetic and parasympathetic regulation. Steinberg alternatively proposes that the heterogeneous loss of atrial innervation due to removal of the anterior fat pad may aggravate heterogeneity of refractoriness, which is important in promoting reentry as the mechanism of AF and is a critical determinant of AF. If so, a number of issues are suggested. One such issue is whether complete denervation might be more effective than preserved innervation. Another such issue is whether there are important detrimental effects on sinus node or AV node function, or other autonomic cardiac responses, when the fat pads are removed.

The interesting property that makes a typical fibrin matrix particularly advantageous for the control of AF in patients recovering from coronary artery bypass graft ("CABG") surgery and other cardiac surgery and procedures is that the typical fibrin matrix is maintained for from only seven to ten days, at which time it begins to degrade. The conduction modification for the typical fibrin matrix therefore is temporary, and may be used to achieve complete denervation during a critical period following the surgery or procedure, followed by a restoration of function to avoid any detrimental effects that may have otherwise resulted from complete irreversible denervation. Even if the SVC-AO fat pad were removed, the remaining RPV fat pad and the IVC-LA fat pad may be treated with a biopolymer to achieve complete denervation during the critical period following the surgery or procedure, followed by a restoration of function in the remaining RPV fat pad and the IVC-LA fat pad.

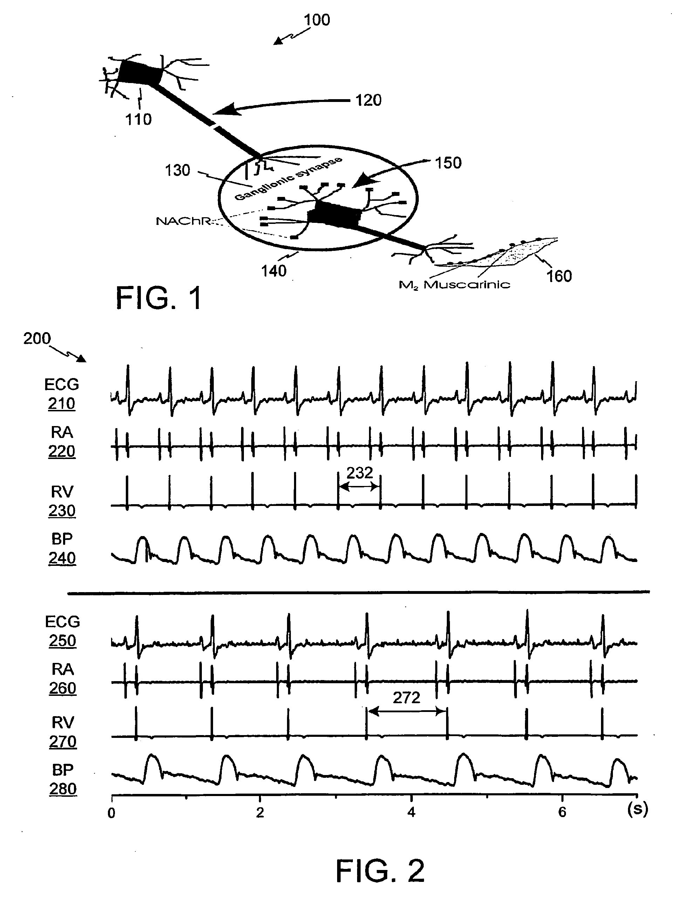

FIG. 1 is a simplified schematic representation showing a nerve pathway 100 from pre-ganglionic neurons 110 in the cervical vagus 120 to sinus node 160. The pre-ganglionic neurons 110 communicate with post-ganglionic neurons 150 via the ganglionic synopses 130 within the RPV or sinus node fat pad 140. The post-ganglionic neurons 150 are coupled to the sinus node 160.

FIG. 2 is a graph 200 showing in the time domain the results after a period of time of injecting fibrin glue into the sinus node fat pad 140 (FIG. 1) of a canine test subject. Time domain traces 210, 220, 230, and 240 represent control data for the sinus rhythm response, and show respectively the ECG, right atrium RA, right ventricle RV, and blood pressure BP signals approximately 4 weeks after injecting the fibrin mixture but prior to applying an electrical stimulus to the cervical vagus 120. Time domain traces 250-280 show the same physiologic responses after an electrical stimulation has been applied to the cervical vagus 120. Taken together, traces 250-280 indicate a significant "slowing down" of the animal's heart rate by the increased time interval between cardiac events. We believe that the fibrin glue matrix reabsorbs so that the electrical stimulation of the cervical vagus 120 has become effective and is able to initiate a parasympathetic response to slow down the animals heart rate.

Another type of conduction-modifying agent is an injectable preparation of fibroblast cells. A fibroblast is a connective tissue cell form the fibrous tissues in the body. We believe that when injected into a fat pad in proximity to a ganglionated plexuses, the fibroblasts engraft in the vicinity of the synapses and mechanically disrupt the conduction of impulses in the synapses. Fibroblasts are electrically insulating and would be expected to inhibits conduction in any electrical synapses that may be present. The effect is persistent.

Another type of conduction-modifying agent is an injectable preparation of fibroblast growth factor ("FGF"). Fibroblast growth factor describes family of cytokines that act on the fibroblasts within the body to induce fibroblast proliferation. Most cells with various organs of the body, including the nervous system and the heart, possess receptors for FGF and therefore are susceptible to its biological effect. Additionally, fibroblast growth factor can be bound to various biopolymers to form a conjugated molecule. Suitable biopolymers include polysaccharides and muco-adhesives. FGF is a small protein that can be easily denatured when exposed to heat or acid. When the FGF is conjugated, the protein is more stable. Conjugation can further program the FGF's release from its carrier in order to ensure that the desired action of the GF, on a specific site, is maintained.

Another type of conduction-modifying agent is an injectable preparation of a neurotoxin. Useful neurotoxins include botulinum toxins such as Botulinum Type A, which is available from Allergan Inc. of Irvine, California under the name BOTOX® Purified Neurotoxin Complex. Another botulinum toxin well known in the art is Botulinum Type B. We believe that when injected into a fat pad in proximity to a ganglionated plexuses, the botulinum toxin disrupts conduction of impulses in the synapses. The effect is temporary, and the neurons generally recover in about three to six months.

The treatment of cardiac arrhythmias according to the principles described herein is considered a highly beneficial non-ablative or minimally-ablative technique for creating conduction blocks in the innervation within the fat pads. This aspect provides immense benefit in providing the intended therapy without many of the other side effects and shortcomings of other conventional techniques for forming cardiac conduction blocks, such as in particular conventional surgical excision and conventional energy ablation. Hyperthermia along with collagen shrinkage and other substantial scarring responses to ablation energy delivery modalities is avoided. Moreover, many ablation techniques suffer from control of energy delivery and extent of impact therefrom in tissues at or beyond the targeted location. For example, many RF energy ablation devices and techniques cause charring, which is associated with the high temperature gradient necessary to form transmural conduction blocks. Undesired energy dissipation into surrounding tissues is often observed using many conventional energy ablation techniques and is also avoided.

The treatment of cardiac arrhythmias according to the principles described herein is relatively easy to carry out, inasmuch as the major fat pads are readily identifiable and the ganglionated plexuses therein are readily accessible. Moreover, the therapeutically effective amount of a conduction-modifying agent is less critical when the agent is applied to the fat pads than when it is applied to other cardiac morphology. The general non-criticality of the therapeutically effective amount is because most types of conduction-modifying agent, including fibroblast, neurotoxin, growth, factor, and biopolymers, become distributed through the fat of the fat pad, including innervated portions in which the desired effect is achieved, as well as in other portions in which they have no or little effect. Fibroblasts additionally multiply in vivo and spread throughout the target tissue.

Preparation Techniques for Some Conduction-Modifying Agents

Fibroblasts may be used to modify conduction in ganglia of the cardiac fat pads. Fibroblasts are normally associated with healing of tissue. Upon activation, a transition of cell types to activated phenotypes occurs. The activated phenotypes having a fundamentally different biologic function from corresponding quiescent cells in normal tissue. These cellular phenotypes (arising from coordinated gene expression) are regulated by cytokines, growth factors, and downstream nuclear targets. They can survive and multiply even in low oxygen environments. Quiescent fibroblasts in normal tissue primarily are responsible for steady-state turnover of the extracellular matrix.

Fibroblast transplantation is used to deliver fibroblast cells to ganglionated plexuses along arrhythmic pathways in the fat pads. The nature of fibroblasts is to fill in space within tissue. When injected into the fat pads, fibroblasts fill in space within the fat pads, but are genetically programmed to cease proliferation and multiplication once they begin to crowd.

Fibroblasts are highly beneficial for creating conduction blocks via cell therapy. In one particular beneficial regard, fibroblasts do not undergo a transition stage from proliferating to mature cells as do skeletal myoblasts when they transform to myotubes. Fibroblasts therefore have a more homogeneous excitation pattern as compared to skeletal muscle. Moreover, the electrophysiological properties of fibroblasts are fairly consistent from one fibroblast to the next, and are believed to be effective for blocking conduction.

An illustrative method of fibroblast preparation is to use autologous fibroblasts from the patient's own body, such as fibroblasts from dermal samples, and subsequently appropriately prepare them (e.g. in a culture/preparation kit) and transplant them to a location within a cardiac tissue structure to retard cardiac tissue conduction along an arrhythmia pathway, to treat conduction disturbances in the heart such as atrial fibrillation, ventricular tachycardia and/or ventricular arrhythmias and CHF. Fibroblasts have the ability to either block or change/remodel the conduction pathway of the heart.

Skin fibroblasts potentiate the migration to PDGF and increase collagen accumulation and MMP synthesis, and net collagen accumulation. This formation of collagen matrix coupled with the lack of gap junction proteins in fibroblasts, creates the potential for electromechanical isolation. A total lack of electrical conduction has been observed in regions with fibroblast migration in the myocardium of patients with a previous myocardial infraction.

Fibroblasts can be biopsied from many tissues in the body (lungs, heart, skin) isolated, amplified in culture, and introduced via injection, graft delivery, grafting, and so forth, either with or without a polymeric carrier or backbone, into a region of the heart where there is a need to modify conduction in or isolate an arrhythmic pathway. Preparations of fibroblasts may include primarily or only one material or combinations of materials. For example, a preparation that include fibroblast cells may also include other materials, such as fluids or other substrates to provide the cells in an overall preparation as a cellular media that is adapted to be injected, such as in particular through a delivery lumen of a delivery catheter. I n one particular example, the fibroblast cells may be combined with a biopolymer material such as fibrin glue, which may itself be provided as two precursor materials that are mixed to form fibrin glue that assists in forming the conduction block when delivered with cells at the desired location within the heart. Collagen or preparations thereof, including precursors or analogs or derivatives of collagen, is also considered useful in such combination.

A biopolymer may be used to modify conduction in ganglia of the cardiac fat pads. The biopolymer is delivered to ganglionated plexuses along arrhythmic pathways in the fat pads. Generally, a polymer is considered to be a chain of multiple units or "mers". Fibrin glue, for example, contains polymerized fibrin monomers, and is further herein considered an illustrative example of a biopolymer since its components are biological. Thrombin in a kit is an initiator or catalyst which enzymatically cleaves fibrinogen into fibrin. The monomers can then polymerize into a fibrin gel or glue. A useful fibrin glue is TISSEAL™, which is available from Baxter Healthcare, Inc. of Chicago, Illinois. Other examples of fibrin glues are disclosed in Sierra, DH, "Fibrin sealant adhesive systems: a review of their chemistry, material properties and clinical applications," J. Biomater Appl., Vol. 7, 1993, pp. 309-52.

The biopolymer may be used alone or in combination with another material. In one beneficial combination, a preparation of fibroblast cells and a biopolymer is delivered into cardiac tissue structures to form a conduction block there. The biopolymer enhances retention of the fibroblast cells being delivered into the location where the conduction block is to be formed, and may also further contribute to forming the conduction block. One particular example of a material that provides significant benefit in such combination with fibroblast cellular therapy is fibrin glue.

Fibroblast growth factor ("FGF") may be used to modify conduction in ganglia of the cardiac fat pads. Growth factors ("GF") generally are small protein chains, commonly known as polypeptides, that bind to cell surface receptor sites and exert actions directly on the target cells. This is generally done through cellular proliferation and or differentiation. Generally, growth factors work at the cellular level to repair damaged cells, enhance cellular proliferation, maintain optimum function of the target organ, and rejuvenate aging tissues.

Fibroblast growth factor describes family of cytokines that act on the fibroblasts within the body to induce fibroblast proliferation. There are perhaps twenty-three members of the FGF superfamily, which interact with at least four distinct types of cell-surface receptors. Fibroblast growth factors are described in further detail in various material from R&D Systems Inc. of Minneapolis, MN, including R&D Systems Inc. 2001 Catalog: Fibroblast Growth Factors, 2001 (http://www.rndsystems.com/asp/g_SiteBuilder.asp?BodylD=308); and R&D Systems Inc. 1996 Catalog: Fibroblast Growth Factor 9, 1996 (http://www.rndsystems.com/asp/g_sitebuilder.asp?bBodyOnly=1 &bodyld=199); see also Gospodarowicz, Denis, Fibroblast growth factor: chemical structure and biologic function, Clinical Orthopedics and Related Research, Number 257, August 1990, pp. 231-248.

Neurotoxin may be used to modify conduction in ganglia of the cardiac fat pads. An example of a useful neurotoxin is botulin, which is any of several potent neurotoxins produced by botulinum and resistant to proteolytic digestion. The mechanism of action of the Botulinum Toxin Type A is disclosed in product information published by Allergan Inc., Botox: mechanism of action (http://www.botox.com/site/professionals/product info/mechanism of action.asp), printed 2004. Essentially, Botulinum toxin type A blocks acetylcholine release by cleaving SNAP-25, a cytoplasmic protein that is located on the cell membrane and that is required for the release of this transmitter.

Various materials described herein are particularly effective and beneficial, such as, for example, fibrin glue and related agents, analogs and derivatives thereof. However, other suitable materials may be used in certain applications, either in combination or as substitutes for such particular materials mentioned. In one particular regard, where fibrin glue or related biopolymer agents are herein described, it is further contemplated that collagen or precursors or analogs or derivatives thereof, may also be used in such circumstances, in particular in relation to modifying conduction in ganglia. Moreover, where collagen is thus included, precursor or analogs or derivatives thereof are further contemplated, such as, for example, structures that are metabolized or otherwise altered within the body to form collagen, or combination materials that reach to form collagen, or material whose molecular structure varies insubstantially to that of collagen such that its activity is substantially similar thereto with respect to the intended uses contemplated herein (e.g., removing or altering non-functional groups with respect to such function). Such a group of collagen and such precursors or analogs or derivatives thereof may be referred to as a "collagen agent." Similarly, other "agents" such as, for example, biopolymer agent, fibrin glue agent, neurotoxin agent, and growth factor agent may further include the actual final product, their precursors separately, or their precursors delivered together or in a coordinated manner to form the resulting material.

While conduction blocks in hearts are provided without substantially abating cardiac tissue, it is appreciated that terms such as "without substantially ablating," "substantially non-ablative," and of similar import are intended to mean that the primary mechanism of action is not ablation of tissue, and that the majority of tissue is not ablated at the location of material delivery. However, it is also to be considered that any material being delivered into a tissue may result in some attributable cell death. For example, the pressure of injection, or even the needle penetration itself, may be responsible for killing some cells, but such is not the mechanism by which conduction block is primarily achieved. In a similar regard, at some level it may be the case that all materials have some toxicity to all cells.

However, a material is herein considered substantially non-ablative with respect to cardiac cells if such material does not substantially ablate tissue as delivered, and cardiac cells can generally survive in the presence of such material in such delivered quantities.

It is also contemplated that cell delivery according to the invention may result in certain circumstances in substantial cell death in, or subsequent apoptosis of, the original cardiac cells in the region of tissue where delivery is performed, but such original cells are replaced by the transplanted cells. The result of such circumstance remains beneficial, as the structure remains cellular as a tissue and considered preferred over a scarred, damaged area as would result from classic ablation techniques.

In addition, despite the significant benefit provided according to the various aspects of the invention for non-ablative conduction blocks, further embodiments may also include ablative modes, such as for example by combining cell or fibrin glue delivery with ablation, either concurrently or serially.

Methods of Delivering Conduction-Modifying Agents to the Fat Pads

A variety of methods may be used to deliver conduction-modifying agents to the ganglia of the fat pads. Suitable methods are described in United States Patent Application Serial No. 10/329,295 filed December 23, 2002 (Randall J. Lee and Mark Maciejewski, System and Method for Forming a Non-Ablative Cardiac Condition Block), International Publication No. WO 03/094855 A1 published November 20, 2003 (Randall J. Lee and Mark Maciejewski, System and Method for Treating Cardiac Arrhythmias with Fibroblast Cells); United States Patent Application Serial No. 10/349,323 filed January 21, 2003 (Randall J. Lee, System and Method for Forming a Non-Ablative Cardiac Conduction Block), and International Publication No. WO 03/095016 A1 published November 20, 2003.

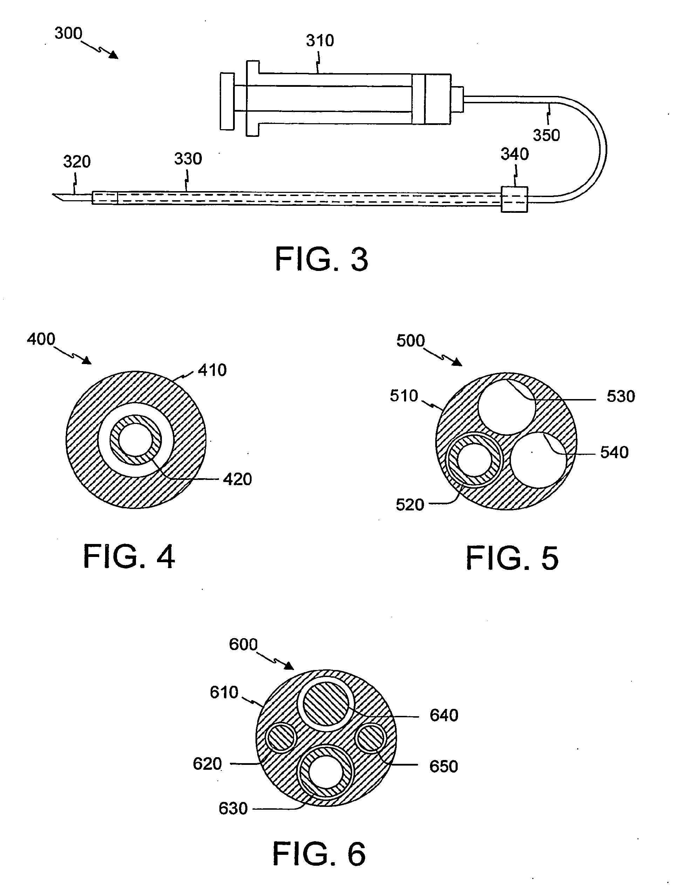

FIG. 3 is a schematic view of an illustrative catheter injection system 300 for a single component conduction-modifying agent. The catheter injection system includes a delivery catheter 330, which is an elongated body that connects at its proximal end by coupler 340 to a tube 350 from the syringe 310, and has a needle 320 at its distal end. A lumen extends through the delivery catheter 330. The needle 320 extends beyond the distal tip of the delivery catheter 330 and into tissue for delivering agent from the syringe 310 into the tissue. The needle 320 may be fixed relative to delivery catheter 330, or may be axially moveable. The needle 320 may take any of a variety of different forms, including the straight sharp-tip type, and the hollow screw-shaped type to aid in anchoring at the target site.

The nature of the delivery catheter 330 may vary considerably depending on the procedure being carried out. FIG. 4 is a transverse cross sectional view of a catheter 400 in which a needle 420 is slideably housed in a single lumen shaft within a catheter body 410. FIG. 5 is a transverse cross sectional view of a catheter 500 in which a needle 520 is slideably housed in a lumen within a catheter body 510. Additional lumens 530 and 540 are provided in the catheter body 510 and may have various different functions, depending upon the particular needs. FIG. 6 is a transverse cross sectional view of a catheter 600 that has four lumens. One of the lumen slideably houses a needle 630, one of the lumen slideably houses pull wire 640, and the remaining two lumens house lead wires 620 and 650, which may be connect to, for example, mapping electrodes. The mapping electrodes are part of a mapping system for detecting an appropriate site for material injection, especially for injection into the fat pads from within the heart (endocardial). Cardiac mapping is well known, and an example is described in Pappone, Carlo et al., Pulmonary vein denervation enhances long-term benefit after circumferential ablation for paroxysmal atrial fibrillation, Circulation 109, r7-r14, 2004.

It will be appreciated that the catheter system 300 of FIG. 3 and the catheter variations shown in FIGS. 4-6 are merely illustrative. Various different types of catheter systems and catheters may be used to deliver and inject conduction-modifying agent into the fat pads.

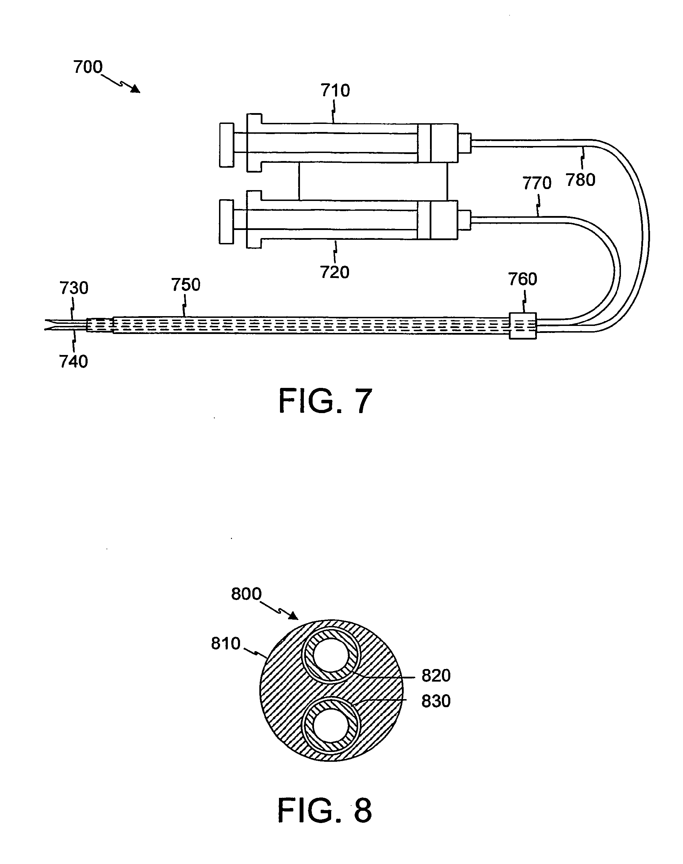

FIG. 7 is a schematic view of an illustrative catheter injection system 700 for a dual component conduction-modifying agent. The catheter injection system includes a delivery catheter 750, which is an elongated body that connects at its proximal end by coupler 760 to two tubes 770 and 780 from respective syringes 720 and 710, and has respective needles 730 and 740 at its distal end. The needles 730 and 740 extend beyond the distal tip of the delivery catheter 750 and into tissue for delivering agent from the syringes 710 and 720 into the tissue. The needles 730 and 740 may be fixed relative to delivery catheter 750, or may be axially moveable.

The catheter injection system 700 is particularly suitable for use with dual component conduction modifying agents for which the components are biopolymer precursors, such as, for example, fibrin glue. When fibrinogen is mixed with thrombin in the presence of calcium ions, it forms fibrin, a filamentous protein that is essential for the clotting of blood. In certain applications, it is desirable to mix fibrinogen and thrombin in a specific region of body tissue, such as the fat pads around the heart (epicardial) or in the heart (endocardial). In general, for many of these applications, it is not desirable to allow the two components to mix other than precisely where they are to be injected. In the catheter injection system 700, the two components are kept entirely separate until the emerge from the distal ends of the needles 730 and 740, so that no opportunity is present for the components to polymerize in the catheter injection system 700 or in any other place than where injected.

FIG. 8 is a transverse cross sectional view of a catheter 800 suitable for the catheter injection system 700. The catheter 800 has two lumens in catheter body 810 which slideably house respective needles 820 and 830. The needles 730 and 740 (FIG. 7) are in fluid communication with the syringes 720 and 710 through the lumens. The fibrin glue precursors flow from their respective syringes to their respective needles along entirely separate channels, so that the precursors mix only after injection into the tissue.

It will be appreciated that the catheter system 700 of FIG. 7 and the catheter variation shown in FIG. 8 are merely illustrative. Various different types of catheter systems and catheters may be used to deliver and inject conduction-modifying agent into the fat pads.

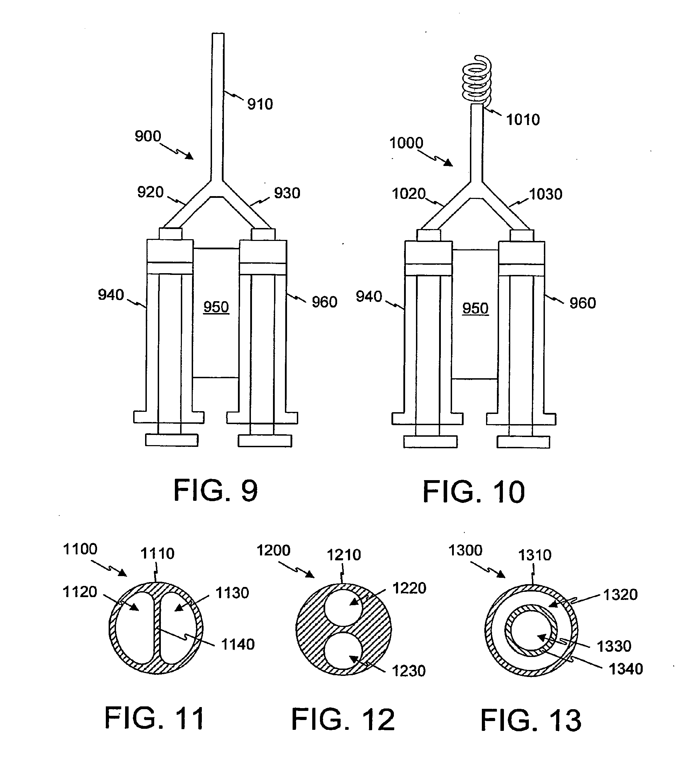

FIG. 9 is a schematic view of an illustrative injection system for injecting a dual component conduction-modifying agent during, for example, open heart surgery. The injection system of FIG. 9 includes two syringes 940 and 960, which may be molded as a single unit or which may be separate syringes held together by clamp 950. The syringes 940 and 960 are shown as having respective hollow barrels fitted with their respective plungers. Alternatively, the plungers from each barrel may be joined together, so that they may be depressed simultaneously. A needle section 900 has a dual channeled portion 910 and branch portions 920 and 930, which are respectively coupled to the syringes 940 and 960. The two components of the conduction-modifying agent from the syringes 940 and 960 are directed through the respective branches 920 and 930 into respective channels in the channeled portion 910. They are kept entirely separate until the emerge from the distal end of the needle section 900, so that no opportunity is present for the components to polymerize in the injector or in any place other than where injected.

FIG. 10 is a schematic view of another illustrative injection system for injecting a dual component conduction-modifying agent during, for example, open heart surgery. The injection system of FIG. 10 is identical to that of FIG. 9, except for differences in a needle section 1000. The needle section 1000 has a helical dual channeled portion 1010 and branch portions 1020 and 1030, which are respectively coupled to the syringes 940 and 960. The two components of the conduction-modifying agent from the syringes 940 and 960 are directed through the respective branches 1020 and 1030 into respective channels in the channeled portion 1010. They are kept entirely separate until the emerge from the distal end of the needle section 1000, so that no opportunity is present for the components to polymerize in the injector or in any place other than where injected.

The needle 900 (FIG. 9) is introduced into the tissue by linear motion, while the needle 1000 (FIG. 10) is introduced into the tissue with a twisting motion and is particularly suitable for moving tissue such as the heart. The needle 900 and the needle 1000 preferably are channeled all the way to their distil tips.

While the injection systems of FIGS. 9 and 10 are dual channeled, any number of separate channels may be provided in the needle. The separate channels of the multi-channeled needle guide the respective precursors to the distal tip of the needle. The tip is injected into a particular region of body tissue, such as the fat pads around the heart or into the heart. The dispensed materials leave the tip, then mix in the injected tissue rather than inside of the needle or the syringe. Any number of barrels may be present on the syringe with a corresponding number of channels extending from the barrels down to the distal tip. The multi-channeled needle may be removed from the barrels for separate disposal, or may be fixed to the hollow barrels. Moreover, the catheter injection systems 300 (FIG. 3) and 700 (FIG. 7) may be provided with multi-channeled needles instead of the single channeled needles shown.

FIGS. 11, 12 and 13 show just a few of the possible way in which dual channels may be provided in a needle. FIG. 11 shows a needle 1100 having a body 1110 that is sectioned with a partition 1140 to form separate and distinct lumens 1120 and 1130. FIG. 12 shows a needle 1200 having a body 1210 within which two separate and distinct lumens 1220 and 1230 are formed. FIG. 13 shows a needle 1300 having a body 1310 that surrounds a second inner body 1340 to form separate and distinct lumens 1320 and 1330. While the lumens and needles are shown as round, they may be oval or any other desired shape.

It will be appreciated that the catheter injection systems and injection systems described herein are illustrative, and other suitable substitutes may be used in order to achieve the objective of delivering two precursor materials and mixing them to form the injected agent. For example, some types of precursor materials may be mixed prior to delivery from the distal portions of the needle, such as at a mixing chamber in proximity to the coupler of the catheter, or prior to coupling to the delivery catheter. Moreover, more than one delivery device may be used for each of two materials being delivered; for example, two separate and distinct needles may be used to deliver each of two precursor materials from respective sources located outside of the patient's body.

Experimental Example

Surgical preparation was as follows. Two adult mongrel dogs (body weight 23-30 kg) were premedicated with thiopental sodium (20 mg/kg) intravenously, and intubated and ventilated with room air supplemented with oxygen as needed to maintain normal arterial blood gases by a respirator (type Narkomed 2, available from North American Drager Inc. of Telford, Pennsylvania). Anesthesia was then maintained with 1-2% isoflurane throughout the experiment. Normal saline solution was infused IV at 100-200 mL/h to replace spontaneous fluid loses. Standard surface ECG leads (I, II, III) were monitored continuously throughout the entire study. Intermittent arterial blood gas measurements were taken and ventilator adjustments were made to correct any metabolic abnormalities. Rectal temperature was monitored with a rectal probe and an electrical heating pad under the animal and operating-room lamps were used to maintain a body temperature of 360C to 370C.

The right femoral artery was cannulated and a micromanometer-tipped catheter pressure transducer (available from Millar, Inc. of Houston, Texas) was inserted and advanced into the thoracic aorta near the aortic valve to monitor systemic blood pressure. After the chest was opened through a median stemotomy, a pericardium cradle was created to support the heart. Custom-made Ag-AgCl quadripolar plate electrodes were sutured to the high right atrium and right ventricular apex for bipolar pacing and recording. Similar bipolar plate electrodes were also used for stimulation of 2 epicardial fat pads that contain parasympathetic neural pathways selectively innervating the sinus node (SN) and the AVN, respectively. The SN fat pad was located at the right pulmonary vein (RPV)-atrial junction. The AVN fat pad was located at the junction of inferior vena cava and the left atrium (IVC-LA). All signals (surface ECGs, right atrial and ventricular electrograms, arterial blood pressure) were amplified, filtered, digitized and continuously displayed on a monitoring system (Prucka Engineering, Inc.). In addition these signals along with calibration signals were simultaneously recorded on magnetic tape (Vetter Digital, 4000A) for later computer analysis with AxoScope (Axon Instruments) and custom software programs.

The study protocol was as follows. The study had 2 stages, initial acute surgery, and observation after 4 weeks recovery. During the initial acute surgery we tested the vagal effects by delivering fat pads' electrical stimulation. The latter was delivered as rectangular pulses at 20 Hz (50 ms interval), 0.2-0.5 ms duration, and amplitude of 3-5 mA. During the final study, in addition to the fat pads, we also delivered electrical stimulation to the cervical vagus (while intact, as well as after decentralization). In this case the parameters were 3, 5 and 10 Hz, 1 ms duration, and amplitude 5 mA.

Fibrin glue was injected during the initial acute study into the 2 fat pads. We used Quixil, a 2-component mixture of thrombin and BAC that was delivered through a 2-channel injector and 23 gauge needle, so that the 2 components mixed only inside the fat pads. The needle was inserted 1-2 mm under the epicardial surface of the fat pads. A total of 1 ml fibrin glue was delivered in each fat pad.

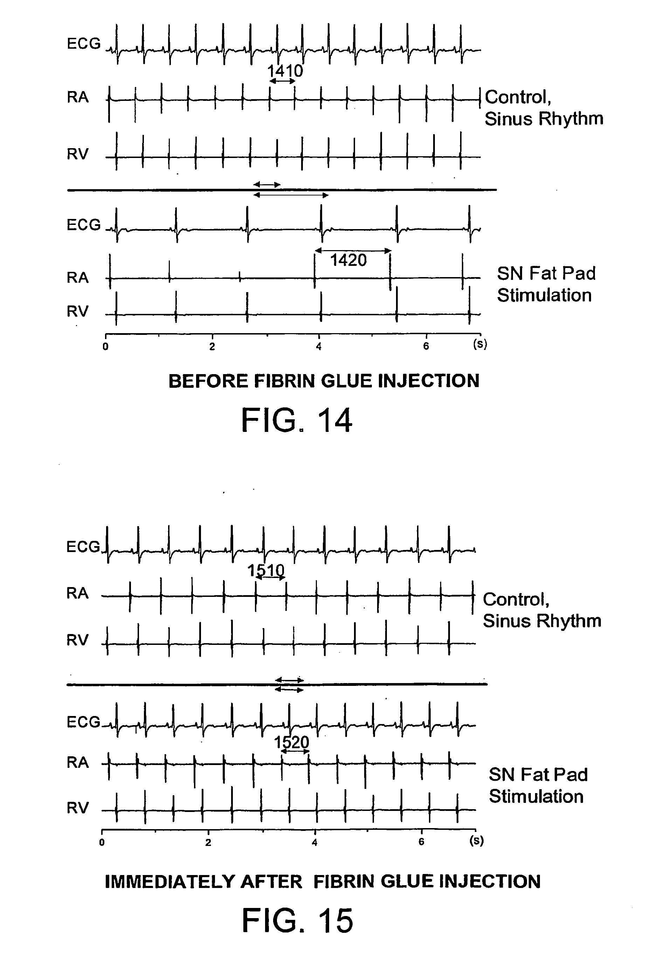

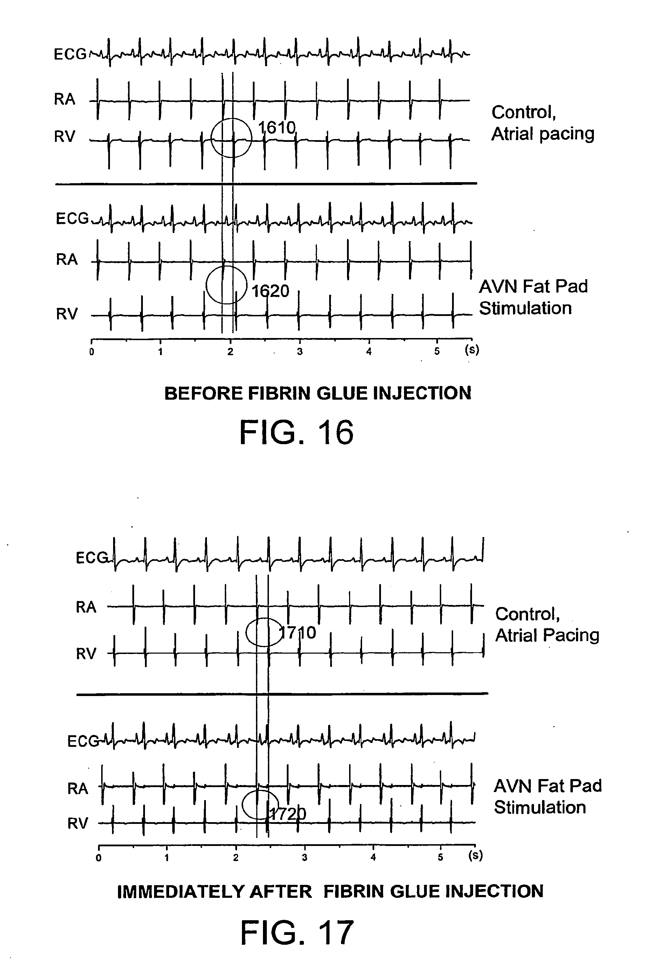

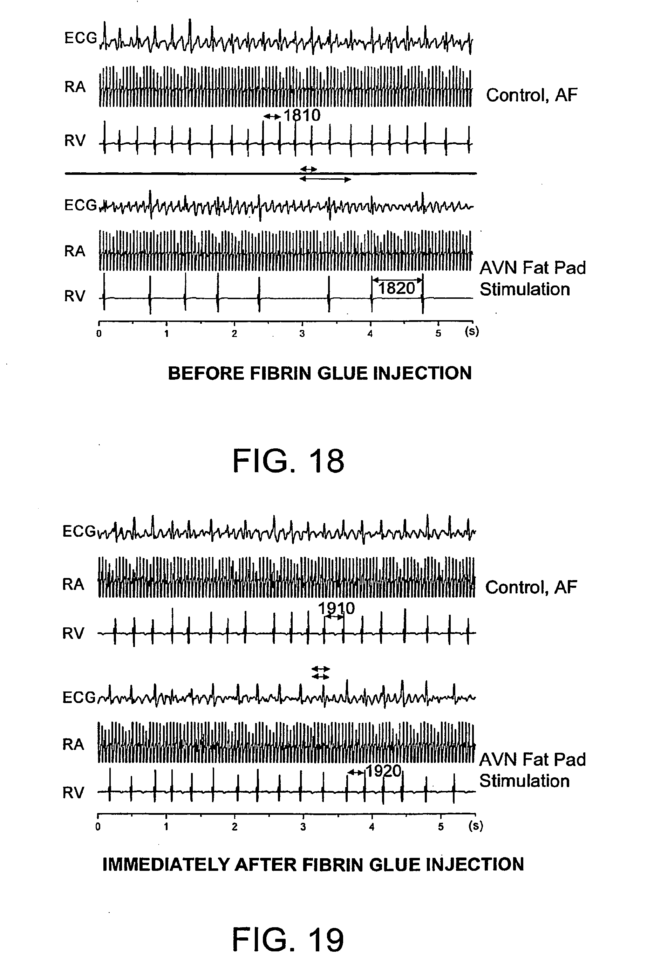

The following results were observed. Electrical stimulation of the fat pads prior to fibrin glue injection resulted in various observable effects. FIG. 14 shows that before fibrin glue injection, electrical stimulation of the sinus node fat pad produced pronounced chronotropic effect; compare interval 1410 with interval 1420 to see prolongation of the cycle length. Similarly, FIG. 16 shows that before fibrin glue injection, electrical stimulation of the AV node fat pad produced clear dromotropic effect; compare relative timing of pulses 1610 and 1620 to see prolongation of the RA-RV interval. Moreover, FIG. 18 shows that during induced atrial fibrillation, the stimulation of the AV node fat pad resulted in strong reduction of the ventricular rate; compare intervals 1810 and 1820. However, these effects were not observed upon electrical stimulation of the fat pads immediately after injection of the fibrin glue. FIG. 15 shows that intervals 1510 and 1520 are essentially equal; compare FIG. 15 with FIG. 14. FIG. 17 shows that the relative timing of pulses 1710 and 1720 is essentially unchanged; compare FIG. 17 with FIG. 16. FIG. 19 shows that intervals 1910 and 1920 are essentially equal; compare FIG. 19 with FIG. 18.

Four weeks after the initial injections the experiments were repeated following the same protocol, and in addition vagal stimulation was also delivered through the cervical vagus. After the 4-week period all vagal effects were present.

## Claims

1. An agent which is effective for modifying conduction of impulses in ganglionic synapses (130) in a cardiac fat pad (140) and substantially non-ablative to the ganglionic synapses (130) in the cardiac fat pad (140), wherein the agent is a biopolymer, fibroblasts, a neurotoxin, or a growth factor, for use in a method for treating cardiac arrhythmia in a heart of a patient, wherein the method comprises injection of a therapeutically effective amount of the agent into at least one cardiac fat pad (140) in proximity to the ganglionic synapses (130).

2. The agent for use according to claim 1, wherein the agent is comprised in a system comprising a cardiac delivery system and a source of the agent coupled to the cardiac delivery system, wherein the cardiac delivery system comprises a distal portion for injecting the agent into at least one cardiac fat pad (140).

3. The agent for use according to claim 1 or 2, wherein the biopolymer is an injectable biopolymer that forms a semi-rigid scaffold upon injection.

4. The agent for use according to claim 1 or 3, wherein the biopolymer is fibrin glue, collagen or alginate.

5. The agent for use according to claim 1, wherein the neurotoxin is Botulinum type A.

6. The agent for use according to claim 1, wherein the growth factor is fibroblast growth factor.

7. The agent for use according to claim 2, wherein the distal portion of the cardiac delivery system comprises at least one needle (320, 420, 520, 630, 730, 740, 820, 830, 900, 1000, 1100, 1200, 1300) having a tip for injecting the agent into proximity with the ganglionic synapses (130) through a surface of the fat pad (140).

8. The agent for use according to claim 7, wherein the needle (320, 420, 520, 630, 730, 740, 820, 830, 900, 1000, 1100, 1200, 1300) is straight.

9. The agent for use according to claim 6, wherein the needle (320, 420, 520, 630, 730, 740, 820, 830, 900, 1000, 1100, 1200, 1300) is helical.

10. The agent for use according to claim 2, wherein the cardiac delivery system comprises an intracardiac delivery system.

11. The agent for use according to claim 2, wherein the cardiac delivery system comprises an endocardial system.

12. The agent for use according to claim 2, wherein the cardiac delivery system comprises a transvascular system for injecting the agent into the fat pad (140) through a wall of a vessel associated with the heart.

13. The agent for use according to claim 2, wherein the cardiac delivery system comprises:
at least one needle (320, 730, 740), the needle (320, 730, 740) having a distal end for injecting the agent into proximity with the ganglionic synapses (130) through a surface of the fat pad (140), and a proximal end; and
a coupler (340, 760) disposed at the proximal end of the needle for coupling the needle (320, 730, 740) to the source.

14. The agent for use according to claim 2, wherein the cardiac delivery system comprises a catheter (300, 700), the catheter (300, 700) comprising:
an elongated body (410, 510, 610, 810, 1110, 1210, 1310) having a proximal end and a distal end;
at least one lumen (530, 540, 1120, 1130, 1220, 1230, 1320, 1330) extending through the elongated body (410, 510, 610, 810, 1110, 1210, 1310) between the distal end of the elongated body (410, 510, 610, 810, 1110, 1210, 1310) and
the proximal end of the elongated body (410, 510, 610, 810, 1110, 1210, 1310);
at least one needle (320, 730, 740) disposed at the distal end of the elongated body (410, 510, 610, 810, 1110, 1210, 1310) and having a tip for injecting the agent into proximity with the ganglionic synapses (130) through a surface of the fat pad (140), the needle (320, 730, 740) being in fluid communication with the lumen (530, 540, 1120, 1130, 1220, 1230, 1320, 1330); and
a coupler (340, 760) disposed at the proximal end of the elongated body (410, 510, 610, 810, 1110, 1210, 1310) for coupling the lumen (530, 540, 1120, 1130, 1220, 1230, 1320, 1330) to the source.

15. The agent for use according to claim 2, further comprising an anchor disposed at the distal portion of the cardiac delivery system for anchoring the distal end of the cardiac delivery system at a location on the fat pad (140) so that the agent may be delivered to a region of tissue in proximity to the location while the anchor is anchored thereto.

16. The agent for use according to claim 2, wherein:
the agent comprises a plurality of components;
the source comprises a plurality of separate sections, the components being respectively separately contained in the source sections; and
the cardiac delivery system comprises a plurality of separate delivery channels, the distal portion of the cardiac delivery system being in fluid communication with the source sections through respectively the delivery channels.

17. The agent for use according to claim 16, wherein the components comprise biopolymer precursors.

18. The agent for use according to of claim 17, wherein the components further comprise fibroblasts, neurotoxin, growth factor, or a combination thereof.

19. The agent for use according to claim 2, wherein the cardiac delivery system comprises an injection needle comprising:
a distal portion comprising a plurality of channels extending to a tip thereof; and
a plurality of separate delivery channels, the distal channels of the cardiac delivery system being in fluid communication with the delivery channels.

20. The agent for use according to claim 19, wherein the distal portion of the injection needle (320, 420, 520, 630, 730, 740, 820, 830, 900, 1000, 1100, 1200, 1300) is straight.

21. The agent for use according to claim 19, wherein the distal portion of the injection needle (320, 420, 520, 630, 730, 740, 820, 830, 900, 1000, 1100, 1200, 1300) is helical.

22. The agent for use according to claim 19 wherein the injection needle (320, 420, 520, 630, 730, 740, 820, 830, 900, 1000, 1100, 1200, 1300) further comprises:
a proximal portion, the delivery channels extending to the proximal portion; and
a coupler (340, 760) for coupling the delivery channels to a catheter (300, 700).

23. The agent for use according to claim 19, wherein the injection needle (320, 420, 520, 630, 730, 740, 820, 830, 900, 1000, 1100, 1200, 1300) further comprises:
a proximal portion, the delivery channels extending to the proximal portion; and
a coupler (340, 760) for coupling the delivery channels to respective syringes (310, 710, 720, 940, 960).

24. The agent for use according to any one of claims 1-23, wherein the agent is used in a method for the treatment of cardiac arrhythmia in a heart of a patient undergoing open heart surgery.

25. The agent for use according to any one of claims 1-24, wherein the agent is injected into at least one cardiac fat pad (140) via at least one target site, the target site being identified by electrical stimulation of the cardiac fat pad (140) in proximity to the ganglionic synapses (130) therein.

26. The agent for use according to any one of claims 1-25, wherein the conduction-modifying agent is injected into the cardiac fat pad (140) with a syringe (310, 710, 720, 940, 960).

27. The agent for use according to any one of claims 1-25, wherein the conduction-modifying agent is injected into the cardiac fat pad (140) with a catheter (300, 700).

28. The agent for use according to claim 7, wherein the needle (320, 420, 520, 630, 730, 740, 820, 830, 900, 1000, 1100, 1200, 1300) is coupled to a syringe (310, 710, 720, 940, 960).

29. The agent for use according to claim 2, further comprising a stimulation delivery system for identifying with electrical stimulation a target site for injecting the conduction-modifying agent into the cardiac fat pad (140).

30. The agent for use according to claim 29, wherein the stimulation delivery system comprises electrodes for the electrical stimulation.

31. The agent for use according to claim 29, wherein the stimulation delivery system further comprises a stimulation generator.

32. The agent for use according to claim 31, wherein the stimulation generator is coupled to the electrodes.

## Patentansprüche

1. Ein Wirkstoff, der für die Modifizierung der Weiterleitung von Impulsen in ganglionären Synapsen (130) in einem kardialen Fettpolster (140) wirksam und für die ganglionären Synapsen (130) in dem kardialen Fettpolster (140) im Wesentlichen nicht-ablativ ist, wobei der Wirkstoff ein Biopolymer, Fibroblasten, ein Neurotoxin oder ein Wachstumsfaktor ist, zur Verwendung in einem Verfahren zur Behandlung von kardialer Arrhythmie in einem Herzen eines Patienten, wobei das Verfahren die Injektion einer therapeutisch wirksamen Menge des Wirkstoffs in mindestens ein kardiales Fettpolster (140) nahe den ganglionären Synapsen (130) umfasst.

2. Der Wirkstoff zur Verwendung gemäß Anspruch 1, wobei der Wirkstoff in einem System umfasst ist, das ein kardiales Verabreichungssystem und eine Wirkstoffquelle, die an das Verabreichungssystem gekoppelt ist, umfasst, wobei das kardiale Verabreichungssystem einen distalen Teil zur Injektionen des Wirkstoffs in mindestens ein kardiales Fettpolster (140) umfasst.

3. Der Wirkstoff zur Verwendung gemäß Anspruch 1 oder 2, wobei das Biopolymer ein injizierbares Biopolymer ist, das bei der Injektion ein halbfestes Gerüst ausbildet.

4. Der Wirkstoff zur Verwendung gemäß Anspruch 1 oder 3, wobei das Biopolymer Fibrin-Klebstoff, Collagen oder Alginat ist.

5. Der Wirkstoff zur Verwendung gemäß Anspruch 1, wobei das Neurotoxin Botulinum Typ A ist.

6. Der Wirkstoff zur Verwendung gemäß Anspruch 1, wobei der Wachstumsfaktor Fibroblasten-Wachstumsfaktor ist.

7. Der Wirkstoff zur Verwendung gemäß Anspruch 2, wobei der distale Teil des kardialen Verabreichungssystems mindestens eine Nadel (320, 420, 520, 630, 730, 740, 820, 830, 900, 1000, 1100, 1200, 1300), die eine Spitze für die Injektion des Wirkstoffs in die Nähe der ganglionären Synapsen (130) durch eine Oberfläche des Fettpolsters (140) hindurch besitzt, umfasst.

8. Der Wirkstoff zur Verwendung gemäß Anspruch 7, wobei die Nadel (320, 420, 520, 630, 730, 740, 820, 830, 900, 1000, 1100, 1200, 1300) gerade ist.

9. Der Wirkstoff zur Verwendung gemäß Anspruch 6, wobei die Nadel (320, 420, 520, 630, 730, 740, 820, 830, 900, 1000, 1100, 1200, 1300) helikal ist.

10. Der Wirkstoff zur Verwendung gemäß Anspruch 2, wobei das kardiale Verabreichungssystem ein intrakardiales Verabreichungssystem umfasst.

11. Der Wirkstoff zur Verwendung gemäß Anspruch 2, wobei das kardiale Verabreichungssystem ein endokardiales System umfasst.

12. Der Wirkstoff zur Verwendung gemäß Anspruch 2, wobei das kardiale Verabreichungssystem ein transvaskuläres System für die Injektion des Wirkstoffs in das Fettpolster (140) durch eine Wand eines Gefäßes, das mit dem Herzen verbunden ist, umfasst.

13. Der Wirkstoff zur Verwendung gemäß Anspruch 2, wobei das kardiale Verabreichungssystem umfasst:
mindestens eine Nadel (320, 730, 740), wobei die Nadel (320, 730, 740) ein distales Ende zur Injektion des Wirkstoffs in die Nähe der ganglionären Synapsen (130) durch eine Oberfläche des Fettpolsters (140) und ein proximales Ende besitzt; und
eine Kopplung (340, 760), die an dem proximalen Ende der Nadel angeordnet ist, um die Nadel (320, 730, 740) mit der Quelle zu koppeln.

14. Der Wirkstoff zur Verwendung gemäß Anspruch 2, wobei das kardiale Verabreichungssystem einen Katheter (300, 700) umfasst, wobei der Katheter (300, 700) umfasst:
einen langgestreckten Körper (410, 510, 610, 810, 1110, 1210, 1310), der ein proximales und ein distales Ende besitzt;
mindestens ein Lumen (530, 540, 1120, 1130, 1220, 1230, 1320, 1330), das durch den langgestreckten Körper (410, 510, 610, 810, 1110, 1210, 1310) zwischen dem distalen Ende des langgestreckten Körpers (410, 510, 610, 810, 1110, 1210, 1310) und dem proximalen Ende des langgestreckten Körpers (410, 510, 610, 810, 1110, 1210, 1310) reicht;
mindestens eine Nadel (320, 730, 740), die an dem distalen Ende des langgestreckten Körpers (410, 510, 610, 810, 1110, 1210, 1310) angeordnet ist und eine Spitze für die Injektion des Wirkstoffs in die Nähe der ganglionären Synapsen (130) durch eine Oberfläche des Fettpolsters (140) besitzt, wobei die Nadel (320, 730, 740) in flüssiger Verbindung mit dem Lumen (530, 540, 1120, 1130, 1220, 1230, 1320, 1330) steht; und
eine Kopplung (340, 760), die an dem proximalen Ende des langgestreckten Körpers (410, 510, 610, 810, 1110, 1210, 1310) angeordnet ist, um das Lumen (530, 540, 1120, 1130, 1220, 1230, 1320, 1330) an die Quelle zu koppeln.

15. Der Wirkstoff zur Verwendung gemäß Anspruch 2, ferner umfassend einen Anker, der an dem distalen Teil des kardialen Verabreichungssystems angeordnet ist, um das distale Ende des kardialen Verabreichungssystems an einer Stelle auf dem Fettpolster (140) zu verankern, sodass der Wirkstoff an eine Geweberegion in der Nähe dieser Stelle verabreicht werden kann, während der Anker daran verankert ist.

16. Der Wirkstoff zur Verwendung gemäß Anspruch 2, wobei:
der Wirkstoff eine Vielzahl von Komponenten umfasst;
die Quelle eine Vielzahl von einzelnen Sektionen umfasst, wobei die Komponenten entsprechend getrennt in den Quellsektionen enthalten sind; und
das kardiale Verabreichungssystem eine Vielzahl von einzelnen Verabreichungskanälen umfasst, wobei der distale Teil des kardialen Verabreichungssystems durch die entsprechenden Verabreichungskanäle mit den Quellsektionen in flüssiger Verbindung steht.

17. Der Wirkstoff zur Verwendung gemäß Anspruch 16, wobei die Komponenten Biopolymer-Vorläufer umfassen.

18. Der Wirkstoff zur Verwendung gemäß Anspruch 17, wobei die Komponenten ferner Fibroblasten, Neurotoxin, Wachstumsfaktor oder eine Kombination davon umfassen.

19. Der Wirkstoff zur Verwendung gemäß Anspruch 2, wobei das kardiale Verabreichungssystem eine Injektionsnadel umfasst, die umfasst:
einen distalen Teil, der eine Vielzahl von Kanälen umfasst, die bis zu einer Spitze davon reichen; und
eine Vielzahl von einzelnen Verabreichungskanälen, wobei die distalen Kanäle des kardialen Verabreichungssystems mit den Verabreichungskanälen in flüssiger Verbindung stehen.

20. Der Wirkstoff zur Verwendung gemäß Anspruch 19, wobei der distale Teil der Injektionsnadel (320, 420, 520, 630, 730, 740, 820, 830, 900, 1000, 1100, 1200, 1300) gerade ist.

21. Der Wirkstoff zur Verwendung gemäß Anspruch 19, wobei der distale Teil der Injektionsnadel (320, 420, 520, 630, 730, 740, 820, 830, 900, 1000, 1100, 1200, 1300) helikal ist.

22. Der Wirkstoff zur Verwendung gemäß Anspruch 19, wobei die Injektionsnadel (320, 420, 520, 630, 730, 740, 820, 830, 900, 1000, 1100, 1200, 1300) ferner umfasst:
einen proximalen Teil, wobei die Verabreichungskanäle bis zu dem proximalen Teil reichen und
eine Kopplung (340,760) zum Koppeln der Verabreichungskanäle an einen Katheter (300,700).

23. Der Wirkstoff zur Verwendung gemäß Anspruch 19, wobei die Injektionsnadel (320, 420, 520, 630, 730, 740, 820, 830, 900, 1000, 1100, 1200, 1300) ferner umfasst:
einen proximalen Teil, wobei die Verabreichungskanäle bis zu dem proximalen Teil reichen; und
eine Kopplung (340, 760) zum Koppeln der Verabreichungskanäle an die entsprechenden Spritzen (310, 710, 720, 940, 960).

24. Der Wirkstoff zur Verwendung nach irgendeinem der Ansprüche 1-23, wobei der Wirkstoff in einem Verfahren zur Behandlung von kardialer Arrhythmie in einem Herzen eines Patienten, bei dem eine offene Herz-Operation durchgeführt wird, verwendet wird.

25. Der Wirkstoff zur Verwendung nach irgendeinem der Ansprüche 1-24, wobei der Wirkstoff in mindestens ein kardiales Fettpolster (140) über mindestens eine Zielstelle injiziert wird, wobei die Zielstelle durch elektrische Stimulation des kardialen Fettpolsters (140) nahe der ganglionären Synapsen (130) identifiziert wird.

26. Der Wirkstoff zur Verwendung nach irgendeinem der Ansprüche 1-25, wobei der leitungs-modifizierende Wirkstoff mit einer Spritze (310, 710, 720, 940, 960) in das kardiale Fettpolster (140) injiziert wird.

27. Der Wirkstoff zur Verwendung nach irgendeinem der Ansprüche 1-25, wobei der leitungs-modifizierende Wirkstoff mit einem Katheter (300, 700) in das kardiale Fettpolster (140) injiziert wird.

28. Der Wirkstoff zur Verwendung nach Anspruch 7, wobei die Nadel (320, 420, 520, 630, 730, 740, 820, 830, 900, 1000, 1100, 1200, 1300) an eine Spritze (310, 710, 720, 940, 960) gekoppelt ist.

29. Der Wirkstoff zur Verwendung gemäß Anspruch 2, ferner umfassend ein Stimulationsverabreichungssystem zur Identifizierung einer Zielstelle für das Injizieren des leitungs-stimulierenden Wirkstoffs in das kardiale Fettpolster (140) mittels elektrischer Stimulation.

30. Der Wirkstoff zur Verwendung gemäß Anspruch 29, wobei das Stimulationsverabreichungssystem Elektroden für die elektrische Stimulation umfasst.

31. Der Wirkstoff zur Verwendung gemäß Anspruch 29, wobei das Stimulationsverabreichungssystem ferner einen Stimulationsgenerator umfasst.

32. Der Wirkstoff zur Verwendung gemäß Anspruch 31, wobei der Stimulationsgenerator an die Elektroden gekoppelt ist.

## Revendications

1. Agent efficace pour modifier la conduction d'impulsions dans des synapses ganglionnaires (130) dans un coussinet adipeux cardiaque (140), et de manière sensiblement non ablative par rapport aux synapses ganglionnaires (130) dans le coussinet adipeux cardiaque (140), lequel agent est un biopolymère, des fibroblastes, une neurotoxine ou un facteur de croissance, destiné à être utilisé dans un procédé de traitement de l'arythmie cardiaque dans le coeur d'un patient, lequel procédé comprend l'injection d'une quantité thérapeutiquement efficace de l'agent dans au moins un coussinet adipeux cardiaque (140), à proximité des synapses ganglionnaires (130).

2. Agent destiné à une utilisation selon la revendication 1, lequel agent est compris dans un système comportant un système d'administration cardiaque et une source de l'agent couplée au système d'administration cardiaque, où le système d'administration cardiaque comprend une partie distale pour injecter l'agent dans au moins un coussinet adipeux cardiaque (140).

3. Agent destiné à une utilisation selon la revendication 1 ou 2, où le biopolymère est un biopolymère injectable qui forme un échafaudage semi-rigide lors de l'injection.

4. Agent destiné à une utilisation selon la revendication 1 ou 3, où le biopolymère est de la colle de fibrine, du collagène ou un alginate.

5. Agent destiné à une utilisation selon la revendication 1, où la neurotoxine est du Botulinum de type A.

6. Agent destiné à une utilisation selon la revendication 1, où le facteur de croissance est un facteur de croissance des fibroblastes.

7. Agent destiné à une utilisation selon la revendication 2, ou la partie distale du système d'administration cardiaque comprend au moins une aiguille (320, 420, 520, 630, 730, 740, 820, 830, 900, 1000, 1100, 1200, 1300) ayant une pointe pour injecter l'agent dans le voisinage des synapses ganglionnaires (130) à travers une surface du coussinet adipeux (140).

8. Agent destiné à une utilisation selon la revendication 7, où l'aiguille (320, 420, 520, 630, 730, 740, 820, 830, 900, 1000, 1100, 1200, 1300) est droite.

9. Agent destiné à une utilisation selon la revendication 6, où l'aiguille (320, 420, 520, 630, 730, 740, 820, 830, 900, 1000, 1100, 1200, 1300) est hélicoïdale.

10. Agent destiné à une utilisation selon la revendication 2, où le système d'administration cardiaque comprend un système d'administration intracardiaque.

11. Agent destiné à une utilisation selon la revendication 2, où le système d'administration cardiaque comprend un système endocardiaque.

12. Agent destiné à une utilisation selon la revendication 2, où le système d'administration cardiaque comprend un système transvasculaire pour injecter l'agent dans le coussinet adipeux (140) à travers une paroi d'un vaisseau associé au coeur.

13. Agent destiné à utilisation selon la revendication 2, où le système d'administration cardiaque comprend :
au moins une aiguille (320, 730, 740), l'aiguille (320, 730, 740) ayant une extrémité distale pour injecter l'agent dans le voisinage des synapses ganglionnaires (130) à travers une surface du coussinet adipeux (140), et une extrémité proximale ; et
un coupleur (340, 760) disposé à l'extrémité proximale de l'aiguille pour coupler l'aiguille (320, 730, 740) à la source.

14. Agent destiné à une utilisation selon la revendication 2, où le système d'administration cardiaque comprend un cathéter (300, 700), le cathéter (300, 700) comprenant:
un corps allongé (410, 510, 610, 810, 1110, 1210, 1310) ayant une extrémité proximale et une extrémité distale ;
au moins une lumière (530, 540, 1120, 1130, 1220, 1230, 1320, 1330) s'étendant à travers le corps allongé (410, 510, 610, 810, 1110, 1210, 1310) entre l'extrémité distale du corps allongé (410, 510, 610, 810, 1110, 1210, 1310) et l'extrémité proximale du corps allongé (410, 510, 610, 810, 1110, 1210, 1310) ;
au moins une aiguille (320, 730, 740) disposée à l'extrémité distale du corps allongé (410, 510, 610, 810, 1110, 1210, 1310) et ayant une pointe pour injecter l'agent dans le voisinage des synapses ganglionnaires (130) à travers une surface du coussinet adipeux (140), l'aiguille (320, 730, 740) étant en communication fluidique avec la lumière (530, 540, 1120, 1130, 1220, 1230, 1320, 1330) ; et
un coupleur (340, 760) disposé à l'extrémité proximale du corps allongé (410, 510, 610, 810, 1110, 1210, 1310) pour coupler la lumière (530, 540, 1120, 1130, 1220, 1230, 1320, 1330) à la source.

15. Agent destiné à une utilisation selon la revendication 2, comprenant en outre un ancrage disposé au niveau de la partie distale du système d'administration cardiaque pour ancrer l'extrémité distale du système d'administration cardiaque à un emplacement sur le coussinet adipeux (140), de sorte que l'agent puisse être administré à une région de tissu à proximité de l'emplacement alors que l'ancrage est ancré à celui-ci.

16. Agent destiné à une utilisation selon la revendication 2, où :
l'agent comprend une pluralité de composants ;
la source comprend une pluralité de sections séparées, les composés étant respectivement contenus séparément dans les sections de source ; et
le système d'administration cardiaque comprend une pluralité de canaux d'administration séparés,
la partie distale du système d'administration cardiaque étant en communication fluidique avec les sections de source à travers les canaux d'administration respectivement.

17. Agent destiné à une utilisation selon la revendication 16, où les composants comprennent des précurseurs de biopolymère.

18. Agent destiné à une utilisation selon la revendication 17, où les composants comprennent en outre des fibroblastes, une neurotoxine, un facteur de croissance ou une combinaison de ces derniers.

19. Agent destiné à une utilisation selon la revendication 2, où le système d'administration cardiaque comprend une aiguille d'injection comprenant :
une partie distale comprenant une pluralité de canaux s'étendant à une pointe de celle-ci ; et
une pluralité de canaux d'administration séparés, les canaux distaux du système d'administration cardiaque étant en communication fluidique avec les canaux d'administration.

20. Agent destiné à une utilisation selon la revendication 19, où la partie distale de l'aiguille d'injection (320, 420, 520, 630, 730, 740, 820, 830, 900, 1000, 1100, 1200, 1300) est droite.

21. Agent destiné à une utilisation selon la revendication 19, où la partie distale de l'aiguille d'injection (320, 420, 520, 630, 730, 740, 820, 830, 900, 1000, 1100, 1200, 1300) est hélicoïdale.

22. Agent destiné à une utilisation selon la revendication 19, où l'aiguille d'injection (320, 420, 520, 630, 730, 740, 820, 830, 900, 1000, 1100, 1200, 1300) comprend en outre :
une partie proximale, les canaux d'administration s'étendant vers la partie proximale ; et
un coupleur (340, 760) pour coupler les canaux d'administration à un cathéter (300, 700).

23. Agent destiné à une utilisation selon la revendication 19, où l'aiguille d'injection (320, 420, 520, 630, 730, 740, 820, 830, 900, 1000, 1100, 1200, 1300) comprend en outre :
une partie proximale, les canaux d'administration s'étendant vers la partie proximale ; et
un coupleur (340, 760) pour coupler les canaux d'administration à des seringues respectives (310, 710, 720, 940, 960).

24. Agent destiné à une utilisation selon l'une quelconque des revendications 1 à 23, où l'agent est utilisé dans un procédé destiné au traitement de l'arythmie cardiaque dans le coeur d'un patient subissant une opération à coeur ouvert.

25. Agent destiné à une utilisation selon l'une quelconque des revendications 1 à 24, où l'agent est injecté dans au moins un coussinet adipeux cardiaque (140) via au moins un site cible, le site cible étant identifié par une stimulation électrique du coussinet adipeux cardiaque (140) à proximité des synapses ganglionnaires (130) présentes dans celui-ci.

26. Agent destiné à une utilisation selon l'une quelconque des revendications 1 à 25, où l'agent modifiant la conduction est injecté dans le coussinet adipeux cardiaque (140) à l'aide d'une seringue (310, 710, 720, 940, 960).

27. Agent destiné à une utilisation selon l'une quelconque des revendications 1 à 25, où l'agent modifiant la conduction est injecté dans le coussinet adipeux cardiaque (140) à l'aide d'un cathéter (300, 700).

28. Agent destiné à une utilisation selon la revendication 7, où l'aiguille (320, 420, 520, 630, 730, 740, 820, 830, 900, 1000, 1100, 1200, 1300) est couplée à une seringue (310, 710, 720, 940, 960).

29. Agent destiné à une utilisation selon la revendication 2, comprenant en outre un système d'administration de stimulation visant à identifier au moyen d'une stimulation électrique un site cible, afin d'injecter l'agent modifiant la conduction dans le coussinet adipeux cardiaque (140).

30. Agent destiné à une utilisation selon la revendication 29, où le système d'administration de stimulation comprend des électrodes pour la stimulation électrique.

31. Agent destiné à une utilisation selon la revendication 29, où le système d'administration de stimulation comprend en outre un générateur de stimulation.

32. Agent destiné à une utilisation selon la revendication 31, où le générateur de stimulation est couplé aux électrodes.
